# DEMANDE DE BREVET EUROPEEN

(11) **EP 1 484 052 A1**
(43) Date de publication de la demande: **08.12.2004**
(21) Numéro de dépôt: 04291256.8
(22) Date de dépôt: 17.05.2004
(51) Int. Cl.: A61K 7/48

(54) **Composition cosmétique comprenant une amine carbonylée**

(30) Priorité: 05.06.2003 FR 0306798
(71) Demandeur: L'OREAL, 75008 Paris (FR)
(72) Inventeur: Cavezza, Alexandre, 93290 Tremblay-en-France (FR); Dalko, Maria, 91190 Gif sur Yvette (FR)
(74) Mandataire: Renard, Emmanuelle

(57) **Abrégé**

La présente invention concerne une composition, adaptée à une application topique sur la peau, comprenant, dans un milieu physiologiquement acceptable, au moins une amine carbonylée de formule (I) :

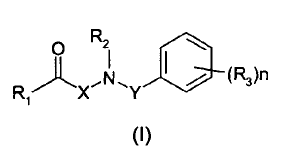

Elle concerne également l'utilisation cosmétique de ce composé, notamment pour prévenir ou lutter contre les signes du vieillissement cutané, notamment pour détendre les traits du visage et/ou lisser les rides, en particulier les rides d'expression.

L'invention concerne également de nouveaux composés de formule (II) :

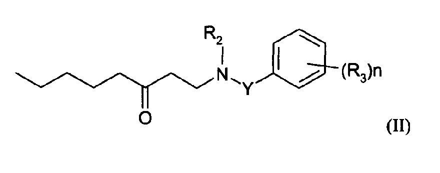

## Description

La présente invention concerne une composition, adaptée à une application topique sur la peau, comprenant, dans un milieu physiologiquement acceptable, au moins une amine carbonylée de formule donnée. Elle concerne également l'utilisation d'une telle amine dans une composition adaptée à une application topique sur la peau, comme agent destiné à détendre les traits du visage et/ou lisser les rides, en particulier les rides d'expression.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en améliorant son renouvellement cellulaire ou encore en favorisant la synthèse, ou en prévenant la dégradation, du collagène.

Bien que ces traitements permettent d'agir sur les rides et ridules dues au vieillissement chronologique et/ou actinique, ils n'ont pas d'effet sur les rides d'expression, lesquelles nécessitent une intervention sur la composante contractile musculaire des rides présente dans la peau.

Jusqu'à présent, le seul moyen couramment utilisé pour agir sur les rides d'expression est la toxine botulique qui est notamment injectée dans les rides de la glabelle qui sont les rides inter-sourcillières (voir J.D. Carruters et al., J. Dermatol. Surg. Oncol., 1992, 18, pp. 17-21).

La Demanderesse a en outre proposé divers composés susceptibles d'offrir un effet dermo-décontractant lorsqu'ils sont appliqués topiquement sur la peau, permettant ainsi d'agir par une autre voie sur les rides d'expression. Parmi ces composés, on peut notamment citer les antagonistes des récepteurs associés aux canaux calciques (FR-2 793 681), et en particulier le manganèse et ses sels (FR-2 809 005) et l'alvérine (FR-2 798 590) ; et les agonistes des récepteurs associés aux canaux chlore, dont la glycine (EP-0 704 210) et certains extraits d'Iris pallida (FR-2 746 641).

Il reste toutefois le besoin de disposer de composés efficaces pour lisser ou estomper les rides d'expression.

Or, la Demanderesse a découvert avec étonnement que certaines amines carbonylées permettaient de satisfaire ce besoin.

Ces amines ont déjà été décrites comme intermédiaires dans la synthèse d'anti-histaminiques (EP-0 209 158), de produits destinés au traitement de la maladie de Parkinson (US-3,009,915), d'anti-inflammatoires (WO 02/083624) et d'anti-spasmodiques (FAUST J. A. et al, *J. Am. Chem. Soc.* (1959), 81, 2214-19). Il a également été suggéré de les utiliser pour promouvoir l'absorption de substances dans le tractus digestif (EP-0 009 609) ou en tant que précurseurs de L-DOPA dans des compositions pharmaceutiques effervescentes (CH-627 077). Elles ont par ailleurs été décrites comme tensioactifs cationiques pour des applications dans le domaine du textile (JP-30 003 216).

Des amines répondant à la définition de celles objet de la présente invention ont en outre été proposées pour une utilisation comme antispasmodiques, destinées à une administration par voie orale, parentérale ou rectale, en vue de relâcher le muscle lisse du tractus gastro-intestinal ou uro-génital ou du système bronchial (US-3,996,245).

Dans le même ordre d'idées, la demande WO 92/04371 divulgue des inhibiteurs de kininogénase, utilisables notamment comme anti-spasmodiques des muscles lisses, dans le traitement de l'asthme ou de l'angine, comprenant des composés proches de ceux objet de la présente invention. Ces composés peuvent notamment être administrés sous forme de compositions appliquées sur la peau.

Ces documents ne suggèrent pas que les composés divulgués pourraient avoir un quelconque effet sur les muscles striés, en particulier sur les muscles peauciers. Or, il est connu dans la littérature que les composés efficaces sur le muscle lisse n'ont pas nécessairement d'effet sur le muscle strié, et vice-versa (American Journal of Veterinary Research (1996), 57(10), 1497-1500 ; Planta Medica (1970), 18(3), 222-6 ; Nippon Yakurigaku Zasshi (1976), 72(1), 41-52 ; Drug Development Research (1992), 25(2), 161-9).

Ainsi, il n'était pas évident que les amines selon la présente invention pouvaient avoir un effet de relaxation des muscles striés permettant d'envisager leur application dans des compositions cosmétiques ou dermatologiques topiques, et en particulier à visée anti-âge.

La présente invention a donc pour objet une composition, adaptée à une application topique sur la peau, comprenant, dans un milieu physiologiquement acceptable, au moins composé de formule (I) : 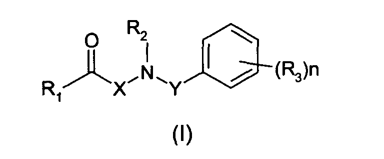 dans laquelle :
R₁ est un groupe alkyle en C₁-C₁₀, linéaire ou ramifié, saturé ou insaturé, éventuellement substitué par un groupe oxo ou par au moins l'un des groupements - OR, -SR, -NRR', -COR, -COOR, -CO-NRR', -NR-CO-R', F et CF₃
où R et R' désignent indépendamment un atome d'hydrogène ou un groupe alkyle en C₁-C₄, linéaire ou ramifié ;
R₂ désigne un atome d'hydrogène ou un groupe alkyle linéaire ou ramifié, saturé ou insaturé, en C₁-C₈, éventuellement substitué par un groupe oxo ;
R₃ désigne
   - un groupe alkyle linéaire, ramifié, saturé ou insaturé, en C₁-C₆, ou un groupe aryle,
   - un groupe -CN, -OR, -SR, -NRR', -COR, -COOR, -CONRR', -NR-CO-R' ou -CF₃, ou
   - un atome d'halogène,
où R et R' ont la signification donnée ci-dessus ;
X est une chaîne alkylène ou alkénylène en C₁-C₉, éventuellement substituée par un groupe oxo ou par au moins l'un des groupements -OR, -SR, -NRR', -COR, -COOR, - CO-NRR', -NR-CO-R' et CF₃ et/ou par un groupe aryle, où R et R' ont la signification donnée ci-dessus ;
Y est une chaîne alkylène ou alkénylène en C₁-C₂₀, éventuellement substituée par au moins l'un des groupements -OR, -SR, -NRR', -COOR, -CO-NRR', -NR-CO-R', F et CF₃ et/ou par un groupe aryle, où R et R' ont la signification donnée ci-dessus ;
n est compris entre 0 et 5,
ou son sel d'addition avec un acide.

Dans la formule (I), les groupes alkyle peuvent notamment être choisis, selon le cas, parmi les groupes : méthyle, éthyle, n-propyle, isopropyle, n-butyle, isobutyle, tert-butyle, pentyle, hexyle, heptyle, octyle, nonyle, décyle, undécyle et dodécyle.

De son côté, le groupe aryle peut être choisi parmi un groupe benzyle et un groupe phényle.

La chaîne alkylène ou alkénylène est en particulier une chaîne éthylène, triméthylène, tétraméthylène, pentaméthylène, vinylène, ou propénylène.

L'atome d'halogène peut être un atome de fluor, de chlore ou de brome.

Comme sels du composé de formule (I), on peut citer les sels obtenus par addition du composé de formule (I) avec un acide inorganique, choisi notamment parmi les acides chlorhydrique, sulfurique, nitrique et phosphorique, ou avec un acide organique, choisi en particulier parmi les acides malonique, succinique, fumarique, lactique, glycolique, citrique et tartrique.

Les composés de formule (I) peuvent notamment être préparés suivant un procédé analogue à celui décrit à l'Exemple 1.

Selon une forme d'exécution préférée de l'invention, le composé de formule (I) est tel que l'une au moins des conditions suivantes est satisfaite :
- R₁ est un groupe alkyle linéaire ou ramifié, saturé ou insaturé, en C₁-C₅,
- R₂ est un atome d'hydrogène ou un groupe alkyle en C₁-C₃,
- R₃ est un groupe alkyle linéaire ou ramifié, saturé ou insaturé, en C₁-C₆,
- n est égal à 0, 1 ou 2,
- X est une chaîne alkylène en C₁-C₄, de préférence une chaîne éthylène,
- Y est une chaîne alkylène en C₁-C₅, de préférence en C₁-C₃.

Plus préférentiellement encore, toutes les conditions ci-dessus sont satisfaites.

Des exemples de composés de formule (I) utiles dans la présente invention comprennent les composés ayant l'une des formules suivantes :

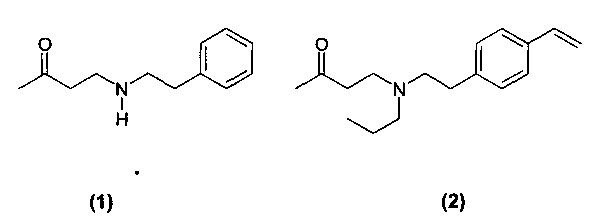

D'autres exemples de composés de formule (I) utiles dans la présente invention comprennent ceux répondant à la formule générale (II) ci-dessous : 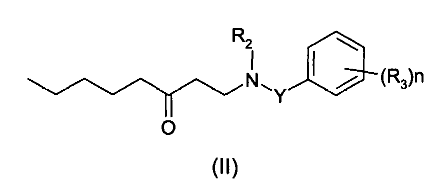 dans laquelle R₂, R₃, n et Y ont la signification indiquée précédemment.

A la connaissance de la Demanderesse, les composés de formule (II) ci-dessus sont nouveaux.

L'invention a donc également pour objet un composé répondant à la formule (II) 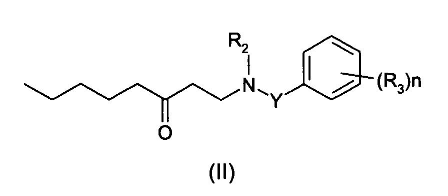 dans laquelle :
R₂ désigne un atome d'hydrogène ou un groupe alkyle linéaire ou ramifié, saturé ou insaturé, en C₁-C₈, éventuellement substitué par un groupe oxo ;
R₃ désigne
   - un groupe alkyle linéaire, ramifié ou cyclique, saturé ou insaturé, en C₁-C₆, ou un groupe aryle,
   - un groupe -CN, -OR, -SR, -NRR', -COR, -COOR, -CONRR' ou -CF₃, ou
   - un atome d'halogène,
où R et R' désignent indépendamment un atome d'hydrogène ou un groupe alkyle en C₁-C₄, linéaire ou ramifié ;
Y est une chaîne alkylène ou alkénylène en C₁-C₂₀, éventuellement substituée par au moins l'un des groupements -OR, -SR, -NRR', -COOR, -CO-NRR', -NR-CO-R', F et CF₃ et/ou par un groupe aryle, où R et R' ont la signification donnée ci-dessus ;
n est compris entre 0 et 5,
ou son sel d'addition avec un acide.

De façon avantageuse, les composés de formule (II) selon l'invention sont tels qu'ils satisfont à l'une au moins des conditions suivantes :
- R₂ est un atome d'hydrogène ou un groupe alkyle linéaire saturé en C₁-C₈, éventuellement substitué par un groupe oxo, de préférence un groupe alkyle en C₁-C₃,
- R₃ est un groupe -OR où R est un atome d'hydrogène ou un groupe alkyle linéaire ou ramifié en C₁-C₄,
- n est égal à 0, 1 ou 2, de préférence 0,
- Y est une chaîne alkylène en C₁-C₅, de préférence en C₁-C₃.

Des exemples de tels composés sont notamment ceux répondant aux formules (3) à (7) ci-dessous :

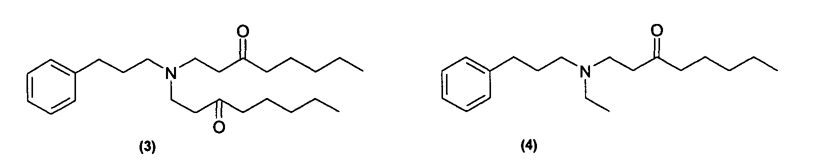

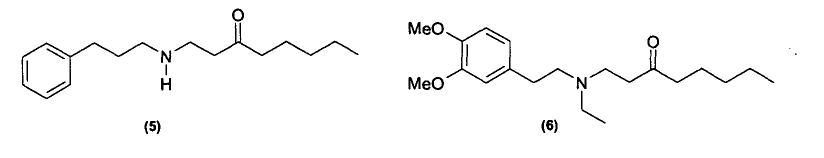

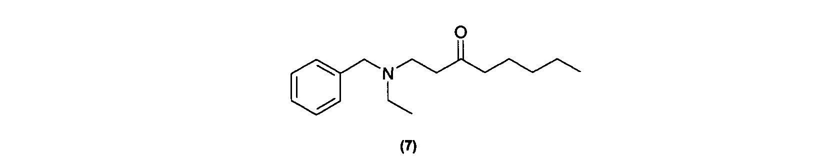

La quantité de composé de formule (I) utilisable selon l'invention est bien entendu fonction de l'effet recherché et peut donc varier dans une large mesure.

Pour donner un ordre de grandeur, on peut utiliser ce composé en une quantité représentant de 0,01% à 10% du poids total de la composition, préférentiellement en une quantité représentant de 0,05% à 5% du poids total de la composition, plus préférentiellement en une quantité représentant de 0,1% à 2% du poids total de la composition.

La composition selon l'invention est adaptée à une application topique sur la peau et elle contient donc un milieu physiologiquement acceptable, c'est-à-dire compatible avec la peau et éventuellement avec ses phanères (cils, ongles, cheveux) et/ou les muqueuses.

Cette composition peut se présenter sous toutes les formes galéniques normalement utilisées dans le domaine cosmétique, et elle peut être notamment sous forme d'une solution éventuellement gélifiée, d'une dispersion du type lotion éventuellement biphasée, d'une émulsion obtenue par dispersion d'une phase grasse dans une phase aqueuse (H/E) ou inversement (E/H), ou d'une émulsion triple (E/H/E ou H/E/H) ou d'une dispersion vésiculaire de type ionique et/ou non ionique. Ces compositions sont préparées selon les méthodes usuelles. On préfère utiliser selon cette invention une composition sous la forme d'une émulsion huile-dans-eau.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sous forme d'aérosol. Elle peut également se présenter sous forme solide, en particulier sous forme de stick. Elle peut être utilisée comme produit de soin et/ou comme produit de maquillage pour la peau.

De façon connue, la composition utilisée selon l'invention peut contenir également les adjuvants habituels dans le domaine cosmétique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans le domaine considéré, et par exemple de 0,01 à 20% du poids total de la composition. Ces adjuvants, selon leur nature, peuvent être introduits dans la phase grasse, dans la phase aqueuse ou dans les vésicules lipidiques. En tout état de cause, ces adjuvants, ainsi que leurs proportions, seront choisis de manière à ne pas nuire aux propriétés recherchées des composés de formule (I) selon l'invention.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Comme huiles utilisables dans l'invention, on peut citer les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras (alcool cétylique), des acides gras, des cires (cire de carnauba, ozokérite).

Comme émulsionnants et coémulsionnants utilisables dans l'invention, on peut citer par exemple les esters d'acide gras et de polyéthylène glycol tels que le stéarate de PEG-100, et les esters d'acide gras et de glycérine tels que le stéarate de glycéryle.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras, la silice hydrophobe et les polyéthylènes.

Comme actifs, la composition selon l'invention peut comprendre au moins un composé choisi parmi : les agents desquamants et/ou hydratants (tels que les α- et β-hydroxyacides, en particulier l'acide n-octanoyl-5-salicylique, les céramides, l'acide hyaluronique, l'acide 2-xothiazolidine-4-carboxylique, l'HEPES, le miel et le glycérol) ; les agents dépigmentants (y compris la vitamine C et ses dérivés tels que le glucoside d'ascorbyle) ; les agents stimulant la prolifération des fibroblastes (dont les extraits de soja) ; les agents stimulant la prolifération des kératinocytes (dont les rétinoïdes tels que le rétinol) ; les agents stimulant la différenciation des kératinocytes (dont les extraits de lupin et de maïs) ; les agents dermo-décontractants (tels que l'adénosine, l'alvérine et les extraits de Wild Yam) ; les agents tenseurs (dont la silice colloïdale, les silicates mixtes et les latex acrylique-silicone) ; et les agents anti-pollution ou anti-radicalaires (dont la vitamine E et le coenzyme Q10).

Comme filtres UV utilisables dans la composition selon l'invention, on peut citer les filtres organiques suivants, cités sous leur nom CTFA ou chimique, selon le cas : Ethylhexyl Salicylate, Ethylhexyl Methoxycinnamate, Octocrylene, Phenylbenzimidazole Sulfonic Acid, Benzophenone-3, Benzophenone-4, Benzophenone-5, 4-Methylbenzylidene camphor, Terephthalylidene Dicamphor Sulfonic Acid, Disodium Phenyl Dibenzimidazole Tetra-sulfonate, la 2,4,6-tris-(4'-amino benzalmalonate de diisobutyle)-s-triazine, Anisotriazine, Ethylhexyl triazone, Diethylhexyl Butamido Triazone, Méthylène bis-Benzotriazolyl Tetramethylbutylphénol, Drometrizole Trisiloxane, 1,1-dicarboxy (2,2'-diméthyl-propyl)-4,4-diphénylbutadiène, et leurs mélanges.

En plus ou à la place des filtres organiques ci-dessus, la composition peut renfermer des agents photoprotecteurs inorganiques choisis par exemple parmi des pigments ou bien encore des nanopigments (taille moyenne des particules primaires: généralement entre 5 nm et 100 nm, de préférence entre 10 nm et 50 nm) d'oxydes métalliques comme par exemple des nanopigments d'oxyde de titane (amorphe ou cristallisé sous forme rutile et/ou anatase), de fer, de zinc, de zirconium ou de cérium. Ces pigments peuvent être ou non enrobés par l'alumine et/ou le stéarate d'aluminium. De tels nanopigments d'oxydes métalliques, enrobés ou non enrobés, sont en particulier décrits dans les demandes de brevets EP518772 et EP518773.

Comme cela sera démontré dans les Exemples ci-après, la Demanderesse a mis en évidence un effet dermo-décontractant des composés de formule (I) selon l'invention, qui permet d'envisager leur utilisation, plus particulièrement dans le lissage des rides d'expression.

L'invention a donc aussi pour objet l'utilisation cosmétique d'au moins un composé de formule (I) ou (II) tel que défini ci-dessus, dans une composition adaptée à une application topique sur la peau, comme agent destiné à prévenir ou lutter contre les signes du vieillissement cutané, notamment comme agent destiné à détendre les traits du visage et/ou lisser les rides, en particulier les rides d'expression.

Elle a encore pour objet un procédé de traitement cosmétique de la peau, notamment d'une peau âgée et plus particulièrement d'une peau ridée, comprenant l'application topique sur ladite peau d'une composition comprenant, dans un milieu physiologiquement acceptable, au moins un composé de formule (I) ou (II) tel que défini précédemment.

Compte tenu de ses propriétés dermo-décontractantes, la composition selon l'invention est avantageusement destinée à être appliquée sur les zones du visage ou du front marquées par des rides d'expression, et/ou sur les personnes présentant des rides d'expression.

Les rides concernées sont de préférence celles disposées radialement autour de la bouche et/ou des yeux, en particulier les rides de la patte d'oie, et/ou situées au niveau du front, en particulier la ride dite du lion, située au niveau de la glabelle, dans l'espace inter-sourcillier, et/ou disposées horizontalement sur le front.

L'invention sera maintenant illustrée par les exemples non limitatifs suivants. Dans ces exemples, les quantités sont indiquées en pourcentage pondéral.

### EXEMPLES

### Exemple 1 : Préparation de la 1-(N-éthyl, (3-phényl-) propylamino) octan-3-one

La 1-(N-éthyl, (3-phényl-) propylamino) octan-3-one peut être préparée suivant un procédé en deux étapes, selon le schéma suivant :

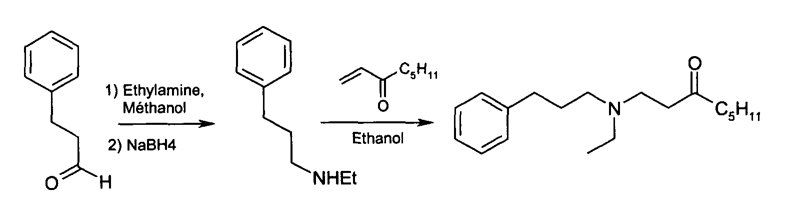

### Etape 1 : synthèse de la N-éthyl, (3-phényl)propylamine

On solubilise 2 g de 3-phényl propionaldéhyde dans 5 ml de méthanol. On ajoute 7,45 g d'éthylamine en solution dans l'éthanol (2 M), puis on laisse réagir pendant 20 heures à température ambiante. On ajoute ensuite lentement 0,68 g de borohydrure de sodium (1,2 eq.) et on laisse agiter pendant 20 heures à température ambiante. Le produit est traité et purifié sur colonne de silice.

On obtient 530 mg de N-éthyl, (3-phényl)propylamine sous forme de produit mono-tache en chromatographie sur couche mince (rendement : 20%). Les résultats de RMN et de spectrométrie de masse sont conformes à la structure attendue.

### Etape 2 : synthèse de la 1-(N-éthyl, (3-phényl-) propylamino) octan-3-one

On solubilise 0,4 g de 1-octène-3-one dans 5 ml d'éthanol absolu. On ajoute ensuite 0,49 g du produit résultant de l'étape 1 en solution dans 5 ml d'éthanol absolu. On laisse réagir pendant 15 heures à température ambiante. Le produit est traité et purifié sur colonne de silice. On obtient 300 mg de produit (rendement : 30%). Les résultats de RMN et de spectrométrie de masse sont conformes à la structure attendue.

### Exemple 2 : Mise en évidence de l'effet dermo-décontractant des composés selon l'invention

Le composé de formule (4) a été testé sur un modèle de co-culture nerfs-muscles qui permet de recréer un arc moteur en innervant des cellules musculaires striées humaines avec des explants de moelle épinière et de ganglions rachidiens d'embryons de rat.

Ce test est prédictif d'un effet anti-rides, comme cela a été mis en évidence par la Demanderesse dans le cas du diazépam, qui inhibait les contractions des fibres musculaires dans ce modèle et dont l'activité anti-rides a été démontrée in vivo.

### a) Protocole

Des cellules musculaires humaines, issues de prélèvements de muscles striés de donneur sain, sont ensemencées dans des puits de 1,8 cm² de section (boîtes de culture de 24 puits). Après 10 jours de culture, ces cellules forment une monocouche et fusionnent. A ce stade, des explants de moelle épinière d'embryons de rat de 13 jours contenant les ganglions rachidiens sont déposées sur la culture.

La croissance des neurites est visible en dehors de l'explant de moelle épinière après un jour de culture. Les premières contractions des fibres musculaires sont observées après 5 à 6 jours de co-culture et après 3 semaines, toutes les fibres musculaires au voisinage des explants se contractent.

Le co-cultures sont utilisées après 21 jours, quand les fibres musculaires sont striées et possèdent des jonctions neuromusculaires différenciées matures.

Une fibre musculaire ayant des contractions régulières (au moins 60 contractions par minute) est alors sélectionnée dans trois puits de culture différents et le nombre de contractions est comptabilisé sur 30 secondes. Le composé testé, dilué à 1/1000 dans le DMSO, est ensuite incubé pendant 60 secondes dans ces puits, à la concentration de 100 µM. A la fin de l'incubation, le nombre de contractions est à nouveau comptabilisé sur 30 secondes. Le test est réalisé en triplicata.

La toxine botulique est utilisée comme référence et testée à 0,1 et 1 µg/ml.

### b) Résultats

Le composé (4) selon l'invention bloque les contractions des trois fibres musculaires référencées à la concentration de 100 µM (0,3 µg/ml).

Par comparaison, la toxine botulique diminue fortement la fréquence des contractions après une minute et bloque complètement les contractions après deux heures d'incubation, à la concentration de 1 µg/ml, et elle a un effet bloquant beaucoup plus faible à une minute, mais également complet après deux heures, à une concentration de 0,1 µg/ml.

Ainsi, les composés selon l'invention sont des agents dermo-décontractants susceptibles d'être utilisés pour détendre les traits du visage et lisser les rides d'expression.

### Exemple 3 : Composition cosmétique

Cette composition est préparée de manière classique pour l'homme du métier. Les quantités indiquées sont en pourcentages pondéraux.

| | |
|---|---|
| Composé de formule (4) | 1 % |
| Isostéarate de propylène glycol | 13 % |
| Polyéthylène glycol (8 OE) | 5 % |
| Propylène glycol | 3 % |
| Pentylène glycol | 3 % |
| Stéarate de glycéryle et stéarate de | |
| polyéthylène glycol (100 OE) | 5 % |
| Mono-stéarate de sorbitane oxyéthyléné (20 OE) | 0,5 % |
| Alcool cétylique oxyéthyléné (20 OE) oxypropyléné (5 OP) | 1 % |
| Gélifiants | 0,5 % |
| Benzoates d'alkyle en C₁₂₋₁₅ | 4 % |
| Ethanol | 3 % |
| Hydroxyde de sodium | 0,12 % |
| Conservateurs | 0,7 % |
| Eau | qsp 100 % |

Ce fluide est destiné à être utilisé en applications mono- ou biquotidiennes sur le visage et le front pour atténuer les rides d'expression.

## Revendications

1. Composition, adaptée à une application topique sur la peau, comprenant, dans un milieu physiologiquement acceptable, au moins composé de formule (I) : 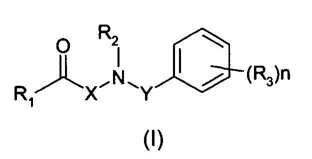 dans laquelle :
R₁ est un groupe alkyle en C₁-C₁₀, linéaire ou ramifié, saturé ou insaturé, éventuellement substitué par un groupe oxo ou par au moins l'un des groupementsOR, -SR, -NRR', -COR, -COOR, -CO-NRR', -NR-CO-R', F et CF₃
où R et R' désignent indépendamment un atome d'hydrogène ou un groupe alkyle en C₁-C₄, linéaire ou ramifié ;
R₂ désigne un atome d'hydrogène ou un groupe alkyle linéaire ou ramifié, saturé ou insaturé, en C₁-C₈, éventuellement substitué par un groupe oxo ;
R₃ désigne
- un groupe alkyle linéaire, ramifié, saturé ou insaturé, en C₁-C₆, ou un groupe aryle,
- un groupe -CN, -OR, -SR, -NRR', -COR, -COOR, -CONRR', -NR-CO-R' ou -CF₃, ou
- un atome d'halogène,
où R et R' ont la signification donnée ci-dessus ;
X est une chaîne alkylène ou alkénylène en C₁-C₉, éventuellement substituée par un groupe oxo ou par au moins l'un des groupements -OR, -SR, -NRR', -COR, -COOR,CO-NRR', -NR-CO-R' et CF₃ et/ou par un groupe aryle, où R et R' ont la signification donnée ci-dessus ;
Y est une chaîne alkylène ou alkénylène en C₁-C₂₀, éventuellement substituée par au moins l'un des groupements -OR, -SR, -NRR', -COOR, -CO-NRR', -NR-CO-R' et CF₃ et/ou par un groupe aryle, où R et R' ont la signification donnée ci-dessus ;
n est compris entre 0 et 5,
ou son sel d'addition avec un acide.

2. Composition selon la revendication 1, **caractérisée en ce que** le composé de formule (I) est tel que l'une au moins des conditions suivantes est satisfaite :
• R₁ est un groupe alkyle linéaire ou ramifié, saturé ou insaturé, en C₁-C₅,
• R₂ est un atome d'hydrogène ou un groupe alkyle en C₁-C₃,
• R₃ est un groupe alkyle linéaire ou ramifié, saturé ou insaturé, en C₁-C₆,
• n est égal à 0 ou 1,
• X est une chaîne alkylène en C₁-C₄,
• Y est une chaîne alkylène en C₁-C₅.

3. Composition selon la revendication 2, **caractérisée en ce que** le composé de formule (I) est tel que X est une chaîne éthylène.

4. Composition selon la revendication 2 ou 3, **caractérisée en ce que** le composé de formule (I) est tel que Y est une chaîne en C₁-C₃.

5. Composition selon l'une quelconque des revendications 1 à 4, **caractérisée en ce que** le composé a l'une des formules suivantes :

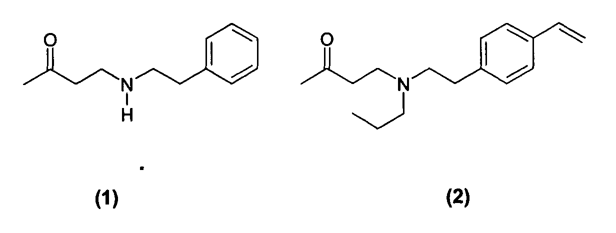

6. Composition selon l'une quelconque des revendications 1 à 5, **caractérisée en ce que** le composé de formule (I) représente de 0,1% à 2% du poids total de la composition.

7. Composé répondant à la formule (II) : 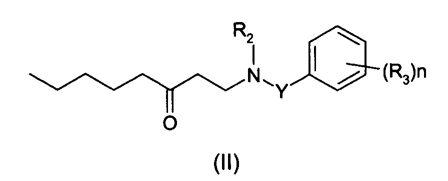 dans laquelle :
R₂ désigne un atome d'hydrogène ou un groupe alkyle linéaire ou ramifié, saturé ou insaturé, en C₁-C₈, éventuellement substitué par un groupe oxo ;
R₃ désigne
- un groupe alkyle linéaire, ramifié ou cyclique, saturé ou insaturé, en C₁-C₆, ou un groupe aryle,
- un groupe -CN, -OR, -SR, -NRR', -COR, -COOR, -CONRR' ou -CF₃, ou
- un atome d'halogène,
où R et R' désignent indépendamment un atome d'hydrogène ou un groupe alkyle en C₁-C₄, linéaire ou ramifié ;
Y est une chaîne alkylène ou alkénylène en C₁-C₂₀, éventuellement substituée par au moins l'un des groupements -OR, -SR, -NRR', -COOR, -CO-NRR', -NR-CO-R', F et CF₃ et/ou par un groupe aryle, où R et R' ont la signification donnée ci-dessus ;
n est compris entre 0 et 5,
ou son sel d'addition avec un acide.

8. Composé selon la revendication 7, **caractérisé en ce qu'**il satisfait à l'une au moins des conditions suivantes :
• R₂ est un atome d'hydrogène ou un groupe alkyle linéaire saturé en C₁-C₈, éventuellement substitué par un groupe oxo,
• R₃ est un groupe -OR où R est un atome d'hydrogène ou un groupe alkyle linéaire ou ramifié en C₁-C₄,
• n est égal à 0, 1 ou 2, de préférence 0,
• Y est une chaîne alkylène en C₁-C₅, de préférence en C₁-C₃.

9. Composé selon la revendication 8, **caractérisé en ce que** R₂ est un groupe alkyle en C₁-C₃.

10. Composé selon la revendication 8 ou 9, **caractérisé en ce que** n est égal à 0.

11. Composé selon l'une quelconque des revendications 8 à 10, **caractérisé en ce que** Y est une chaîne alkylène en C₁-C₃.

12. Composé selon la revendication 7 ou 8, **caractérisé en ce qu'**il répond à l'une des formules ci-dessous :

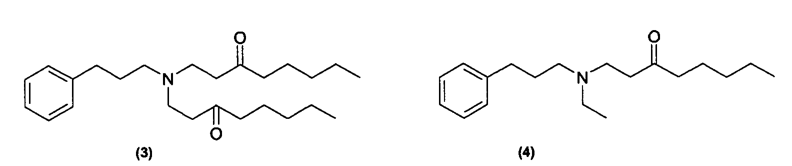

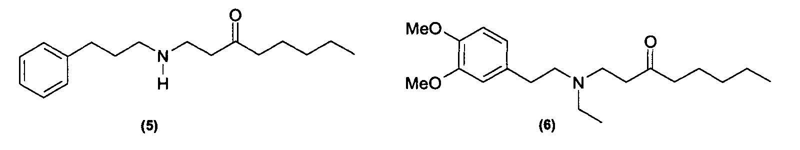

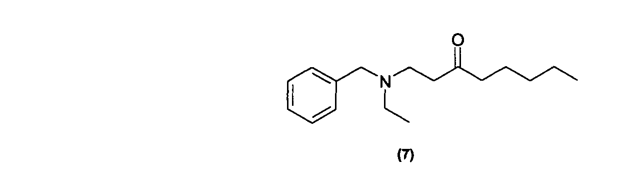

13. Utilisation cosmétique d'au moins un composé de formule (I) défini dans l'une quelconque des revendications 1 à 5 ou d'un composé de formule (II) selon l'une quelconque des revendications 7 à 12, dans une composition adaptée à une application topique sur la peau, comme agent destiné à prévenir ou lutter contre les signes du vieillissement cutané.

14. Utilisation selon la revendication 13, **caractérisée en ce que** ledit composé est utilisé comme agent destiné à détendre les traits du visage et/ou lisser les rides.

15. Utilisation selon la revendication 14, **caractérisée en ce que** lesdites rides sont des rides d'expression.

16. Procédé de traitement cosmétique de la peau, comprenant l'application topique sur ladite peau d'une composition comprenant, dans un milieu physiologiquement acceptable, au moins un composé de formule (I) ou (II) tel que défini dans l'une quelconque des revendications 1 à 5 et 7 à 12.

17. Procédé selon la revendication 16, **caractérisé en ce que** ladite peau est une peau âgée.

18. Procédé selon la revendication 16, **caractérisé en ce que** ladite peau est une peau ridée.

19. Procédé selon la revendication 16, **caractérisé en ce que** ladite composition est appliquée sur les zones du visage ou du front marquées par des rides d'expression et/ou sur les personnes présentant des rides d'expression.

20. Procédé selon la revendication 16, **caractérisé en ce que** ladite composition est appliquée sur les rides disposées radialement autour de la bouche et/ou des yeux et/ou horizontalement sur le front et/ou situées dans l'espace inter-sourcillier.
